# EUROPÄISCHE PATENTSCHRIFT

(11) **EP 0 889 053 B1**
(45) Veröffentlichungstag und Bekanntmachung des Hinweises auf die Patenterteilung: **19.10.2005**
(21) Anmeldenummer: 98250225.4
(22) Anmeldetag: 22.06.1998
(51) Int. Cl.: C07K 14/47, C07K 1/14, A61K 35/12, A61P 17/06, A61P 35/00, A61P 29/00

(54) **BK-RiV-Präparate zur Behandlung von proliferativen Zellerkrangungen**
BK-RiV preparations for teatment of proliferative cellular disorders
Préparations à base de BK-RiV pour le traitement de maladies prolifératives cellulaires

(30) Priorität: 27.06.1997 DE 19728323
(43) Veröffentlichungstag der Anmeldung: 07.01.1999
(73) Patentinhaber: Solisch, Peter, dr.med.habil., 17498 Riemserort (DE)
(72) Erfinder: Solisch, Peter, Dr.med.habil., 17498 Riemserort (DE); Kalies, Kai-Uwe, Dr., 10405 Berlin (DE); Bergmann, Steffi, Dr., 10247 Berlin (DE)
(74) Vertreter: Ziebig, Marlene

(56) Entgegenhaltungen:
- EP-A- 0 702 955
- DD-A- 156 667
- DE-A- 4 238 766
- DE-U- 9 218 127
- SOLISCH: "Zur Dignität einer bisher unbekannten Zellreaktion: "RiV". Eine Hypothese" ZENTRALBLATT FÜR PATHOLOGIE, Bd. 138, Nr. 2, 1992, Seiten 136-140, XP002083610
- COX J C ET AL: "Adjuvants--a classification and review of their modes of action" VACCINE, Bd. 15, Nr. 3, Februar 1997, Seite 248-256 XP004094403

## Beschreibung

Die Erfindung betrifft die Verwendung von BK-RiV zur Herstellung von Präparaten für die Medizin und Veterinärmedizin zur Therapie und Prophylaxe von Zellerkrankungen unter Stabilisierung des "Allgemein zellulär-sekretorischen Abwehrsystems" (AZSA) und der mit diesem kausal verknüpften Körperzustände.

Es wurde bereits in der Patentschrift DD 228 739 vorgeschlagen, BK-RiV - bestehend aus zellulärpartikulären Resistenzfaktoren und hergestellt aus kultivierten, speziell stimulierten eukarionten Zellen - als Therapeutikum gegen Virus- und Tumorerkrankungen einzusetzen. Weiterhin wurde ein Verfahren zur Herstellung von BK-RiV mit Hilfe speziell selektierter Zellinien in der Patentschrift DD 283 330 vorgeschlagen. In der Patentschrift DD 227 327 wurde die Gewinnung des Therapeutikums aus Vollblut und in der Patentschrift DD 268 159 die Verwendung des Therapeutikums als Stimulus für Zellkulturen zur Induktion von ebenfalls therapeutisch wirksamen Stoffen vorgeschlagen. Neue Erkenntnisse hinsichtlich der Inhaltsstoffe und der Herstellungstechnologie des Therapeutikums wurden im Gebrauchsmuster G 92 18 127.9 vorgeschlagen. Weitere Vorschläge im Zusammenhang mit dem Therapeutikum betrafen die Verwendung desselben als diagnostisches Antigen und die Nut-zung der gegen dieses gerichteten Antikörper in der Patentschrift DE 42 38 766 sowie die Anwendung des Therapeutikums gegen AIDS jeder Ätiologie in der Patentschrift EP 0 702 955.

Die vorgeschlagenen Verfahren und Anwendungen beruhten auf der praktischen Ausnutzung der Entdeckung der "Zellreaktion in Vertebratenzellen" (RiV). Sie bezogen sich auf einzelne Aspekte der Anwendung dieser Entdeckung. Ein möglicher Zusammenhang mit einer breiten Anwendung in der Humanmedizin, Veterinärmedizin und Biologie als Leitgedanke für ein theoretisches Konzept, vor allem für eine breitere Anwendung der Präparate, war nicht voraussehbar.

Ziel der Erfindung ist es, anwendbare Präparate zu offenbaren für den vorbeugenden, den heilenden und den lindemden Zweck in der Human - und Veterinärmedizin bei Zellerkrankungen durch molekularbiologisch bedingte, für das Gesamtindividuum schädliche (Fehl-) Informationen, die die Störung oder die Zerstörung des in der Evolution festgelegten Musters des jeweils betroffenen Organs durch pathologische Proliferation von veränderten Körperzellen (Träger der Fehlinformation) verursachen und bei denen das AZSA nicht in der Lage ist, den Vorgang in physiologischer Weise zu beenden, wodurch folglich in komplizierten Wechselwirkungen mit der Gegenreaktion des Gesamtorganismus in zahlreichen Einzelfällen jeweils definierte, an sich bekannte, unterschiedliche Krankheitsmanifestationen entstehen, sowie Verfahren zu ihrer Herstellung aufzuzeigen.

Der Erfindung liegt die Aufgabe zugrunde, Präparate bereitzustellen, die Mensch und Tier vor dem Wirken fremder, schädlicher, biologischer Informationen, die in den Körper eindringen können, eingedrungen oder in ihm entstanden sind, schützen, d.h. das AZSA substituieren.

Es wurde gefunden, daß Seren großer Probandengruppen von gesunden Menschen, insbesondere von Blutspendern, wie in wiederholten Studien nachgewiesen wurde, zu etwa 20 % positive BK-RiV-Antikörper aufwiesen, was der Tumorhäufigkeit der mitteleuropäischen Erwachsenen entspricht. Bekannt ist dabei, daß die Entwicklung der Manifestation einer Tumorerkrankung länger als 5 Jahre währen kann und bereits Tumore der Ausdehnung im mm-Bereich, die mit den üblichen normalen Methoden der Tumorerkennung nicht ermittelt werden können, Tumorzellen in das Blut abgeben. Bei der intravenösen Dauerinfusion von BK-RiV sind sowohl eine "Virusreinigung des Serums" als auch eine "massive intracorporale Tumorzellbeseitigung" in kurzem Zeitintervall bekannt, ebenso die allgemeinen bzw. lokalen Grundwirkungen - insbesondere im prophylaktisch-physiologischen Bereich - bei enteraler sowie externaler Applikation. Es wurde nun weiterhin gefunden, daß extreme morphinresistente Schmerzzustände unterschiedlicher Ätiologie durch 2 ml BK-RiV im Zeitintervall von 15 min bis zu drei Tagen p.a. völlig aufgehoben und in eine positive Stimmungslage umgewandelt werden können. Besonders überraschend war die Feststellung, daß Mykosen, insbesondere Nagelmykosen durch BK-RiV-Therapie ohne Zusatzbehandlung zum Verschwinden gebracht werden konnten, sowie daß eine generalisierte Psoriasis, d.h. generalisierte psoriatische Erythrodermie ohne Zusatztherapie zur weitgehenden Rückbildung gebracht werden konnte.' Durch eine BK-RiV-Therapie bei zeitgleicher oder phasenverschobener Anwendung von Chemo- oder Radiotherapie konnten deren toxische Nebenwirkungen einerseits und deren erwünschte Wirkung andererseits positiv beeinflußt werden und Zellschädigungen bei dem Auftreten einer Virus-Hepatitis kurzfristig aufgehoben werden, was jedoch nicht Gegenstand der Erfindung ist. Im Rahmen der Erfindung konnte jedoch außerdem festgestellt werden, daß bei der RiV-Therapie allergische Erkrankungen, wie z.B. eine Sonnenallergie und Heuschnupfen sofort beendet wurden. Weiterhin wurde gefunden, daß sich positive Heilungsverläufe bzw. Verbesserungen des Krankheitsbildes bei Plasmozytom und malignem Histiozytom (Sarkom) ergaben.

Gegenstand der Erfindung ist deshalb die Verwendung von BK-RiV zur Herstellung von Präparaten für die Medizin und Veterinärmedizin zur Therapie und Prophylaxe von Zellerkrankungen durch molekularbiologisch bedingte, für das Gesamtindividuum schädliche (Fehl-) Informationen, die die Störung oder die Zerstörung des in der Evolution festgelegten Musters des jeweils betroffenen Organs durch pathologische Proliferation von veränderten Körperzellen verursachen und bei denen das "Allgemein zellulär-sekretorische Abwehrsystem" (AZSA) nicht in der Lage ist, den Vorgang in physiologischer Weise zu beenden, sowie zur Stabilisierung und Substitution des AZSA und der mit diesem kausal verknüpften Körperzustände, wobei die BK-RiV-Präparate
a) zur Behandlung von extremen Schmerzzuständen,
b) bei der Behandlung von Mykosen,
c) zur Behandlung von Psoriasis,
d) zur Behandlung von allergischen Erkrankungen,
e) zur Behandlung von Plasmozytom oder
f) zur Behandlung von (Sarkom) Histiozytom
eingesetzt werden.

Es konnte nach molekularbiologischen und biochemischen Prüfungen festgestellt werden, daß BK-RiV hochwirksame Mitglieder mehrerer Proteinfamilien enthält, die im Sinne vorliegender experimenteller Untersuchungen zusammenfassend folgende Effekte nach Applikation hervorrufen: antientzündlich, virodestruktiv, apoptoseauslösend für virusinfizierte oder tumoröse Zellen, stimulierend für das zelluläre und humorale Immunsystem, antimykös, zellprotektiv, antiallergisch, schmerzaufhebend, psychisch positiv stimulierend, chemotaktisch regulierend, signalübertragend. In den BK-RiV-Päparaten wurden folgende Proteine, assoziiert mit RiV-Partikeln, nachgewiesen: Annexin V, Annexin 1, 14-3-3 ξ, 14-3-3 ε, Galectin-3, S100A4, S100A2, Glutathion-S Transferase, Nm23, Aldolase, gCAP39, Myosin (leichte Kette), Actin, Vimentin, Nucleolin, α-Actinin, Histon 2B, Histon 2 A, ribosomales Protein L7, HSP27, HSP70, Proteasom C6, Proteasom (δ), Proteasom (1), Ezrin, ER-60, PDI, Enolase, natural killer enhancing factor, zum Pflanzenprotein Hevein (Lectin) homologes Säugerprotein (Teilsequenz: XQXGRQAGGKLXPDN) und zu Stratherin homologes Protein (ca. 50 k Da, Teilsequenz: FGYGYGPYQPVPEQP), Annexin-I-Isoform (H 156 Y). Die drei letztgenannten Proteine sind bisher nicht beschrieben worden. Sie sind neu und in Verbindung mit den anderen Proteinbestandteilen des BK-RiV als physiologisch wirksame Verbindungen anzusehen, die in der Diagnose, Prophylaxe und Therapie eingesetzt werden. Die Proteine sind jedoch nicht Gegenstand der vorliegenden Erfindung. Es wurde nachgewiesen, daß nicht-virale RNA ein wesentlicher Bestandteil von BK-RiV ist und sie die räumliche Assoziation der RiV-Proteine als Strukturelement und -stabilisator gewährleistet und daß sie zur schonenden Herstellung von BK-RiV genutzt werden kann. Die Komplexwirkung aller unversehrten Bestandteile von BK-RiV stellt für die bisher festgestellten Effekte:
Unschädlichkeit und spezielle Wirksamkeit die kausale Ursache dar. Die Unversehrtheit der RiV-Partikel ist Bedingung für die Unschädlichkeit und Wirksamkeit der BK-RiV-Therapie, denn die wirksamen Proteine bedürfen der engen räumlichen Assoziation mit der Partikelstruktur von RiV, da in fast jedem Fall eine eigene Proteinsignalsequenz zur Sekretion fehlt und nur für einige Proteine Zelloberflächenrezeptoren nachgewiesen wurden, aber bei der RiV-Partikel-Struktur die Exo- und Endozytose als gegeben betrachtet werden kann. Dies gilt auch für RiV-Subpartikel.

Zur extremen Schonung, d.h. der Bewahrung der natürlichen Unversehrtheit der RiV-Partikel-Struktur ist ein geschlossener computergesteuerter Produktionsprozeß von BK-RiV unumgänglich. Dieser Produktionsprozeß eröffnet gleichzeitig mit einer erhöhten RiV-Partikel-Ausbeute den Weg zur Großproduktion und beinhaltet folgende Merkmale: Produktionstemperatur: etwa 4 - 8°C; automatisiert: Zellauswahl aus Masterpool; Aussaat, Kultivierung unter Verwendung von Fermentoren, Ernte durch automatische Bilderkennung und Zellzählung; maßgerechte Zellresuspension in isotonischer, pH-neutraler anorganischer Pufferlösung;

Ultraschalldestruktion der Zellen unter computergesteuerter Sichtkontrolle bis zum notwendigen Verschwinden von Zell- oder Zellbestandteilaggregationen; klärende Zentrifugation durch automatisch kontrollierte Klarheit des Überstandes; Ultrazentrifugation des Überstandes nach vorgegebenen Kriterien; Resuspendierung des Ultrasediments mit anschließender Klärungszentrifugation; definierte Chloroformbehandlung in Schüttelmaschine mit anschließender Klärzentrifugation, Restchloroformbehandlung unter Anwendung eines inerten Gases; Gewinnung des Überstandes; wesentliche Vorbestimmung und Stufenkontrolle der Produktion, elektronenmikroskopische Sichtkontrollen (eventuell durch automatisch-programmierte Bilderkennung): Kriterium: Menge und Erhaltungszustand der BK-RiV-Partikel und Reinheit d.h. Freiheitsgrad von unerwünschten Begleitgebilden. BK-RiV weist sowohl hinsichtlich der Herstellung der therapeutisch einsetzbaren BK-RiV-Präparationen
als auch hinsichtlich des ärztlichen Einsatzes eine große Vielfalt auf. Ein großer Wirkungsbereich besteht für Erkrankungen, die sich aus Störungen des AZSA und durch pathologische Zeltproliferationen ergeben und in Anspruch 1 charakterisiert sind. Das betrifft die Besonderheiten der Speziesspezifität, d.h. weitgehende Speziesunspezifität der BK-RN-Präparation und die Therapie von Virusinfektionen sowie von zellproliferativen Erkrankungen im weitesten Sinne der allgemeinen Pathologie und Erkrankungen, die einer Verbesserung bzw. Gesundung des AZSA und damit der Immunantwort bedürfen. Nach Prüfung von BK-RiV mit modernsten Verfahren weist es keine unphysiologischen, d.h. schädlichen Proteine bzw. virale oder onkogene DNA und/oder RNA oder andere gefährliche Stoffe auf, d.h. RiV ist eine sinnvoll-positiv-funktionelle Reaktion der Vertebratenzelle zum Schutze gegen bereits früh in der Evolution aufgetretene pathogenetische und ätiologische Prozesse, die der Gesundheit entgegenstanden und entgegenstehen. Der ärztliche Einsatz von BK-RiV als isoliertes zelluläres Sekretionsprodukt, das eine morphologisch und molekularbiologisch definierte hochgereinigte Präparation darstellt, gewährleistet, daß sich mittels seiner komplexen Wirkung, die in der Natur vorgegebenen, körperliche Strukturen und Prozesse insbesondere des Immunsystems als Ganzheit physiologisch entfalten. Damit wird eine prinzipiell neue Grundordnung in der Medizin und Veterinärmedizin erkennbar, die die gedankliche Brücke zwischen monokausalem Denken und dem Streben nach ganzheitlichem Verständnis der genannten angewandten Wissensdisziplinen schlagen kann.

Durch eine Zerlegung des BK-RiV in Einzelkomponenten wird es ermöglicht, die einzelnen Bestandteile, die erhalten werden, chemisch und pharmakologisch zu charakterisieren und der gentechnischen Herstellung zugänglich zu machen, um isoliert zur praktischen Nutzanwendung gegen einzelne Krankheiten eingesetzt zu werden. Dabei können die wirksamen Komponenten mit spezifischen Bindungsstellen für Zellobemächenrezeptoren zur Bindung an die Oberflächen spezifischer Zellen bzw. zum Passieren der Zellmembranen versehen werden.

### Beispiele

1. (nicht Gegenstand der Erfindung)
   Die Seren Gesunder, wie z.B. Blutspender und aus statistischen Gründen Tumorgefährdeter, werden im indirekten ELISA (Enzym-Immunassay) auf Antikörper gegen BK-RiV geprüft. Bei einem BK-RiV-Antikörper-Nachweis erfolgt eine kurzfristige Kontrolle innerhalb von vier Wochen, bei Persistenz derselben die Erhebung des klinischen Status zur Eingrenzung einer möglichen Ursache. Erfolgt die Feststellung einer wahrscheinlichen Tumorgenese, muß entsprechend der biologischen und sozialen Situation eine mögliche Haupttumorlokalisation statistisch ermittelt und Diagnostik durchgeführt werden. Betroffene Blutspender sind bis zur Klärung der Situation vom Spenden auszuschließen. Bei persistierenden hohen BK-RiV-Antikörper-Reaktionen und statistisch zu erwartender hoher Tumorwahrscheinlichkeit sollte - auch ohne Tumordiagnose - eine BK-RiV-Therapie durchgeführt werden.
2. (nicht Gegenstand der Erfindung)
   Patient A. geboren 1941; seit etwa fünf Jahren progredienter Verlauf einer "Spondylitis hyperostotica" bei "arterieller und konsekutiver pulmonaler Hypertonie, KHK"; starke Zunahme der Beschwerden: erhebliche Gliederschmerzen, Gewichtsabnahme, Inappetenz, mechanische Atembeeinträchtigung, allgemeine Schwäche mit fast völliger Unfähigkeit für banale Selbstversorgung; nach Bitten des Patienten BK-RiV-Behandlung: am 17.06., 18.06., 19.06., 23.07., 24.07., 02.08., und 23.09.1996 jeweils 1 bzw. 2 ml. Vergleich ausgewählter Immunzellen im peripheren Blut: 1. unmittelbar vor erster Applikation; 2. zwei Tage p.a.; 3. fünf Tage p.a.; 4. fünf Wochen p.a.; 5. sechs Wochen p.a.; 6. dreizehn Wochen p.a.: (Zellanzahl in GPt/l) NK-Zellen: 0,28; 0,37; 0,41; 0,44; 0,35; 0,29; Helferzellen: 0,58; 0,50; 0,62; 0,55; 0,55; 0,66; Suppr./zytot. Zellen: 0,45; 0,50; 0,52; 0,50; 0,48; 0,58; Antistreptolysin-O-Titer 233 lU/ml am 17.06.1996,
   156 lU/ml am 02.08.1996; 171 IU/ml am 23.09.1996; unmittelbar p.a. Beginn der Wirkung: Min.: Leichtigkeit und unwahrscheinlicher Optimismus, 7 Std.: aufrechter Gang möglich, "längeres Rasenmähen", "Gefühle des besseren Atmens, keine Schmerzen mehr, bessere Beweglichkeit der HWS (im Auto Umdrehen des Kopfes möglich), Appetit, Gewichtszunahme, starke Erhöhung der körperlichen Leistungsfähigkeit (8 Std. Arbeit möglich)".
   Epikrise: Bei schwerem progredienten Verlauf einer Spondylitis hyperostotica war die Applikation von BK-RiV mit Aufhebung der Schmerzen, der größten Bewegungseinschränkungen, der mangelhaften Leistungsfähigkeit und der schlechten Stimmungslage für Monate verbunden. Der Antistreptolysin-Titer normalisierte sich, die NK-Zellen vermehrten sich signifikant, die T8- und die T4-Zellen zeigten tendenzielle Erhöhungen. Endergebnis: weitgehende Normalisierung der Lebensqualität.
3. Patientin B. geboren 1949 (Ärztin für Neurologie, Psychiatrie, Psycho- und Schmerztherapie); Ovarialcarzinom, multiple Metastasierung; Ausschöpfung aller Möglichkeiten: Chirurgie, Chemotherapie; Finalphase; schwere gegen alle bisherigen Möglichkeiten resistente Schmerzzustände: insbesondere der Metastase im Hüftgelenk re.; Bitte um RIV-Behandlung als ultima ratio durch behandelnde Ärzte einer Universitätsstadt; seit 29.09.1996 in Tagesabständen BK-RiV jeweils 2 ml; mehrere Patientenberichte unter ausdrücklicher Berücksichtigung erworbener professioneller Erfahrung zur Schmerztherapie; am 18.10.1996: "Wenige Minuten p.a. Gefühl der Hinfälligkeit und schweren Asthenie verschwunden; Stimmungswechsel von subdepressiv-besorgt zu optimistisch, fröhlich, unternehmungslustig; Denkabläufe wendiger, viel schneller, negative Informationen bringen die Patientin nicht mehr aus dem Gleichgewicht; vor Injektion: Siechtum und massive Suizidgedanken; danach Appetit prompt, Plagen durch Hungergefühl; stechende Schmerzen re. Hüfte deutlich rückläufig; drei erhabene Neurofibrome der re. Wange deutlich rückläufig; stärkste Wirkung: drei Tage; 4. und 5. Tag etwas geringer; 6. und 7. Tag deutliche Abnahme; am 8. Tag war das Befinden so schlecht wie vor der Applikation;" am 15.11.1996: "Bereits 10 min. p.a. Abnahme der Schwere und Kraftlosigkeit der Glieder; Erhöhung der geistigen Beweglichkeit; Stimmung optimistisch; innere Körperwärme macht sich breit; Schmerzen im Hüftgelenk deutlich weniger: das Bein kann zum hinkenden Gang angesetzt werden; Appetit, leichte Gewichtszunahme; ab 4. und 5. Tag: Abnahme der Wirkung; am 6.Tag: keine Wirkung mehr, dann massive Verschlechterung (7., 8. und 9. Tag); am 19.11.1996: nach Applikation am 18.11.1996 (2 ml) erstmals seit der Operation völlig schmerzfrei; am 09.12.1996: "Bericht idem"; 20.12.1996: fortschreitendes Finalstadium: Wirkung von 2 ml BK-RiV auf drei Tage begrenzt. Mündliche Beurteilung der Patientin.: "kein bisher bekanntes Schmerzmittel hat eine so gute, anhaltende Wirkung ohne unerwünschte Nebeneffekte - es ist das Mittel der Wahl im genannten Anwendungsbereich".
   Epikrise: Bei generalisierten metastasierendem Ovarial-Carzinom waren extreme Schmerzzustände und depressive Stimmungslagen mit 2 ml BK-RiV bei bisher dreimonatiger Anwendung jeweils für mehr als drei Tage völlig beherrschbar.
4. (nicht Gegenstand der Erfindung)
   Bei den AIDS-Patienten: C. geboren 1959; D. geboren 1964; E. geboren 1965 u.a. gelang es durch BK-RiV-Therapie in zweistelliger ml-Gesamtmenge innerhalb von Monatsfristen, die HIV-RNA um 6 Zehnerpotenzen auf unterhalb der Nachweisgrenzen zu senken, bei gleichzeitiger Verbesserung klinischer und labortechnischer nachweisbarer Parameter.
5. Durch BK-RiV-Therapie gelang es bei dem Hautarzt, Dr. F. geboren 1943, eine Fußnagelmykose ohne weitere Begleittherapie, die jahrelanger Selbstbehandlung trotzte, zu heilen (die BK-RiV-Behandlung erfolgte wegen eines Tumorleidens), idem bei Patient G. geboren 1965 (die RiV-Behandlung erfolgte hier wegen AIDS).
6. (nicht Gegenstand der Erfindung)
   Bei den Patienten H. geboren 1949; 1. geboren 1934; J. geboren 1923 mit ausgedehnten intraabdominellen Carzinomen wurde während der BK-RiV-Therapie (etwa 1 Jahr) eine Femmetastasenbildung nicht beobachtet.
7. (nicht Gegenstand der Erfindung)
   Bei den Carzinom-Patienten: K. geboren 1929; L. geboren 1940; M. geboren 1941 und bei den AIDS-Patienten N. geboren 1965 und O. geboren 1966 konnten durch gleichzeitige oder phäsenverschobene BK-RiV-Therapie die toxischen Nebenwirkungen der Chemotherapie aufgehoben oder sehr stark gemildert werden und eventuell sogar eine Potenzierung der erwünschten Wirkung erreicht werden.
8. Patient P. geboren 1961; AIDS durch HIV-Infektion, Zusammenbruch des Immunsystems; ausgelöst durch Antibiotika: generalisierte psoriatische Erythrodermie, großflächige Schuppungen im Sinne des Lyell-Syndrom ("Syndrom der Verbrühten Haut"), Köbner-Phänomen (tautropfenartige Blutungen); Photodokumentation; Therapie: zweitägig 5 mg Neotigason; Kachexie, starke allgemeine Schwäche und Müdigkeit; Bitte um BK-RiV-Behandlung als ultima ratio; vom 05.08.1996 bis 01.11.1996 insgesamt 21 ml BK-RiV; vergleichende Labordaten (vom 07.06.1996) vor und während BK-RiV-Behandlung (vom 10.09.1996): Gesamt-Lymphozyten: 530 und 1158; Monozyten: 240 und 580; "Mature-T-Lymphozyten: 140 und 210; Cytotoxic/Suppressor T-Zellen: 110 und 180; Helfer/Inducer: 0 und 4 cu/mm; Befund vom 29.11.96 (in %): Normalisierung aller Immunzellen bis auf CD4 (2,8 %; Mangel) und T-Cytotox (65,2 %, stark überschießend); telefonische Mitteilung des Patienten am 01.12.1996: "Psoriasis nur an den Waden; AZ wesentlich verbessert: Aktivität; Gewichts- und Appetitzunahme"; am 01.11.1996 bereits Photodokümentation der Haut: "leichtes Psoriasis-Exanthem mit seborrhoisch-dysseborrhoischem Aspekt und pityriasiformer Schuppung (kleieartig), streifige Entzündungskriterien: Abheilphase"; am 18.12.1996 Mitteilung der Dermatologin: nach Absetzen der RiV-Behandlung und Beginn von Protease-Hemmer-Applikation Verschlechterung der Psoriasis.
   Epikrise: Bei AIDS durch HIV, Zusammenbruch des Immunsytems, Kachexie, generalisierte psoriatische Erythrodermie, AZ eines Schwerkranken war die Applikation von 21 ml BK-RiV innerhalb von drei Monaten assoziiert mit signifikanter Anhebung der Anzahl von Immunzellen im peripheren Blut und weitgehendem Abklingen (praktisch: Ausheilung) der lebensbedrohenden Komplikation "generalisierte Psoriasis-Erytrodermie" und mit deutlicher Verbesserung des AZ.
9. Nach BK-RiV-Therapie konnte bei Patientin Q. geboren 1949 (Behandlung wegen einer rheumatischen Erkrankung) eine schwere Sonnenallergie sofort beendet werden, ebenso akuter Heuschnupfen bei Patienten R. geboren 1941, der wegen eines Rectumcarzinoms behandelt wurde.
10. Patientin S. geboren 1944; 1993 Diagnose: Plasmozytom (TypOlgA Lambda) mit generalisiertem Knochenbefall; anfangs Rückbildung der Hypercalziämie und der erheblichen Knochenschmerzen nach Therapie Aredia, Alexan, Alkeran und Decortin (mehrere Zyklen); folgend Erhaltungstherapie mit Interferon Alpha (wöchentlich 1x): schlechte Verträglichkeit, Abbruch; Knochenmarksbiopsie: monomorphes Bild von Plasmazellen, ausgeprägte Knochenmarksinvasion, erhebliche fast generalisierte Knochenschmerzen; vermutlich plasmazellulläre Infiltration der Niere: Myelomniere mit Niereninsuffizienz, Erhöhung der Kretinin- und Kalziumserumspiegel; Intensivierung der Therapie durch VAD-Zyklus angeraten, von Patientin abgelehnt; Alkeran intravenös, Aredia-Infusionen, ergänzt durch Osmofundin, Einzelgaben von Cortison und Erypo: dadurch Verlangsamung der zunehmenden Anämie, Stabilisierung der Thrombozytenzahlen auf über 100 000 pro ml; dennoch in drei-wöchentlichen Intervallen Bluttransfusion notwendig; Frühjahr 1995 wiederholte Anfragen zur und Bitten um BK-RiV-Behandlung: Ultima ratio obwohl bisher keine einschlägigen Erfahrungen; vom 08.05. bis 29.05.1995 insgesamt 13 ml BK-RiV i.m. appliziert ("Monotherapie"), beginnen mit 1 ml am 1.Tag, 2 ml je am 2. und 3.Tag, nach 1 Woche idem usw. in abfallenden Dosen; Laborkontrollen (10.05., 15.05., 24.05., 24.05., 30.05.1995 und persönliche Mitteilung des Hausarztes vom 21.06.1995): Kreatinen, Kalzium, Status des peripheren Blutes, einschließlich des Immunstatus, RiV-Test; klinische Kontrolle des AZ; geringer Anstieg des Kreatinin 188 pmol/l, 208, 220, 224; Stabilisierung mit nichtsignifikanter absteigender Tendenz ("positive Stabilisierung") des Kalzium: 2,69 mmol/l, 2,64; 2,59; 2,67; ebenfalls "positive Stabilisierung" folgender weißen peripheren Blut-Zellen-Subpopulationen: absolute Zellzahlen: T8-Lymphozyten: 0,39 GPUI, 0,49; 0,42; T4-Lymphozyten: 0,21 GPt/I; 0,26; 0,23; natürliche Killerzellen: 0,21 GPt/l, 0,26, 0,21; aktivierte T-Zellen: 15 %, 18 %, 18 %; Gesamtlymphozyten: 0,48 GPUI; 0,58; 0,49; Monozyten: 0,8 GPUI; 0,9; 0,10; 0,10; 0,10; am 21.06.1995 allen T-Lymphozyten-Subpopulationen normal; Veränderung des Quotienten T4/T8 von 0,5 auf 0,6: Absinken der B-Lymphozyten absolut von 0,10 auf 0,01 GPUI, der Erythrozyten von 3,1 TPUI auf 2,4; des Hb von 6,20 mmol/l auf 4,8; der Thrombozyten: 122 GPt/l, 112, 84, 90; RiV-Antikörper waren nicht nachweisbar am: 12.05., 16.05. und 01.06.1995; persönlicher Bericht der Patientin, 28.05.1995 schriftlich, von Juni mündlich: Besserung des AZ: 35 Km-Radtour, erhebliche Schmerzlinderung, Verbesserung der Stimmungslage, der körperlichen Leistungsfähigkeit, obwohl "Zeit wäre für Bluttransfusion, subjektiv keine notwendig"; "eindeutig Verbesserung der Lebensqualität"; Reisefähigkeit mit Gepäck, allein, Bundesbahn; in folgenden Monaten: wiederholte dringliche Bitten um Weiterbehandlung; entgegenstehend: nicht gelöstes Transportproblem; September: Verschlechterung des AZ.
   Epikrise: Unter BK-RiV-Behandlung Stabilisierung von T8-, T4-Lymphozyten- und aktivierten Lymphozyten- und der Monozyten-Anzahl sowie der natürlichen Killerzellen bei langsamen Abfall der übrigen Blutzellanzahl, auch der B-Lymphozyten; Stabilisierung des Serum-Kalzium-Spiegels; wesentliche Verbesserung der Lebensqualität (Stimmungslage, Schmerz, Leistungsfähigkeit); keine unerwünschten Nebenwirkungen weder lokal noch allgemein.
11. Patientin T. geboren 1945; Z. n. "Knochen-Tbc" li. Oberschenkel in Kindheit, Z. n. Fraktur daselbst (Autounfall vor 10 Jahren), seit April 1995 Schmerzen, Schwellung daselbst mit Fieber, Hausärztin: "V. a. Tumor": am 29.04.1995 Blutentnahme für RiV-Test: IgG 0,245 E (1:10), 0,156 E (1:50), IgM Normwert; bildgebende Diagnostik und am 02.05.1995 Operationsdiagnose: Hämatom; am 12.05.1995 auf Initiative der Hausärztin Re-Op. m. Histologie: "Histiozytom"; am 18.05.1995 Blutentnahme für RiV-Test: IgG 0,532 E (1:10), 0,507 (1:50), IgM 0,250 E (1:10), 0,155 E (1;50); am 16.06.1995 radikale Tumor-Op. mit Rekonstruktionsplastik (Amputation von Patientin abgelehnt); am 11.07.1995 Probeneingang zum RiV-Test: IgG 0,490 E (1:10), 0,523 E (1:50), IgM 0,306 E (1:10), 0,136 E (1:50); Chemotherapie von Patientin abgelehnt; am 17.09.1995 Mitteilung der Hausärztin: "seit 3 Wochen Bestrahlung"; am 18.09.1995 Blutentnahme für RiV-Test: IgG 0,388 E (1:10), 0,381 E (1:50), IgM 0,127 E (1:10), 0,078 E (1:50); starke Schmerzen LWS; Prognose-Mitteilung eines Klinikarztes an die Patientin: "Weihnachten werden Sie nicht erleben"; Bitte der Hausärztin um BK-RiV-Behandlung: am 16.10., 17.10 und 18.10.1995 insgesamt 5 ml BK-RiV: sehr gut vertragen, AZ verbessert, weitgehende Besserung der Schmerzen, Antritt einer Überseereise; Absinken der BSG: vor BK-RiV-Behandlung 50/70, danach am 23.12.1995: 4/10 mm; Verbesserung des Immunstatus des peripheren Blutes unmittelbar vor und 8 Wochen nach BK-RiV-Behandlung (23.12.1996): absolut (µl): Leukozyten: 5.110 und 6.100; Lymphozyten: 870 und 1.460; Monozyten:360 und 430; Granulozyten: 3.880 und 4.210; T-Zellen gesamt: 660 und 860; T4-Helfer/Induktor-Zellen: 440 und 630; T8-Suppr./Zytot.Zellen: 220 und 260; akt. T-Zellen: 100 und 160; Leu-2+7+Zellen: n. u. und 160; MHC-unrestricted CTL: 40 und 40; B-Zellen: 80 und 150; NK-Zellen: 100 und 290; T4/T8-Quotient: 2,04 und 2,39; Jan. 1996 briefliche Mitteilung der Hausärztin: "Sehr gutes Allgemeinbefinden". Jan. 1996: RiV-Antikörper gleichbleibende hohe Werte (Impfeffekt). November 1996 keine Verschlechterung bekannt.
   Epikrise: Schmerzen im li. Oberschenkel führten zum Verdacht der Hausärztin auf Tumorerkrankung. Der RiV-Test war positiv. Bildgebende Diagnostik und Operation ergaben die Diagnose: Hämatom. In der Folge wurde weiterer Anstieg der Extinktion im RiV-Test beobachtet, Gewebsentnahme bei erneuter Operation, ergab die Diagnose: "Histiozytom (Sarkom)". Nach Tumorausräumung kam wiederum Anstieg der RiV-Antikörper im Serum zur Feststellung, Wochen danach langsamer Abfall derselben. Mit BK-RiV-Behandlung wurde als ultima ratio begonnen und war in der Folge assoziiert mit: wesentlicher Verbesserung des Allgemeinbefindens, Normalisierung des Immunstatus im peripheren Blut sowie der Blutsenkungsgeschwindigkeit. Eine Überseereise wurde von der Patientin absolviert.
12. Der extrem die RiV-Partikel schonende, für die Großproduktion geeignete BK-RiV-Herstellungsprozeß hat folgende Charakteristika: In GMP-gerechten, gekühlten Räumen (4 bis 8 °C) beginnend mit archivierungsgerechter Lagerung (flüssiger Stickstoff) des Mastercellpools, Kennzeichnung, Abfüllung, Reihenfolgenfestlegung der Verwendung, Wiederarchivierung nach Anzüchtung zum Erhalt des Mastercellpools) durch programmgesteuerte Automatik; protrahierte Kultivierung der Aussaat mittels definierter Zellmenge des Masterpools in Rollerflaschen oder Fermentoren unter Kontrolle von Bilderkennungssystemen, Zellemte nach Signalen des Bilderkennungssystems, Gewinnung des Zellsedimentes und Resuspendierung, Ultraschalldestruktion aller Zellelemente außer RiV-Partikel unter Kontrolle der Homogenität der Lösung durch Bilderkennungssystem (Trübung, optische Dichte), Klärungszentrifugation, Gewinnung des Überstandes, Ultrazentrifugation und Gewinnung des Sedimentes automatisiert nach vorgegebenen, an sich bekannten Verfahren, Resuspension des Ultrasedimentes in anorganischer, pH-neutraler Pufferlösung; die Kriterien der Computerprogramme: elektronenmikroskopische Stufenkontrolle des Erhaltungszustandes, der Menge und der Freiheit von fremden Morphen der RiV-Partikel-Präparationen; weitere automatisierte Klärungszentrifugation, programmierte Chloroform-Behandlung mit Zentrifugation und maschinelle Abnahme des Überstandes: Endzustand des BK-RiV-Präparates mit automatischer Vorprüfung (Proteingehalt u.a.).
13. (nicht Gegenstand der Erfindung)
   Klonierung und Sequenzierung des bisher unbekannten zu Hevein homologen Proteins - Aminosäureteilsequenz: XQXGRQAGGKLXPDN - aus BK-RiV, Überführung mit Hilfe an sich bekannter gentechnischer Verfahren in die molekularbiologische Synthese mit hohem Reinheitsgrad, Einsatz als "Antibiotikum" gegen Pilzerkrankungen des Menschen, der Tiere und der Pflanzen, sowie des bisher unbekannten zu Stratherin homolgen Proteins: Aminosäuresequenz: FGYGYGPYQPVPEQP; sowie die neue Isoform des Annexin 1 (HI 56Y).
14. Klonierung von NDKB (Nm23) aus BK-RiV, Überführung mit Hilfe an sich bekannter gentechnischer Verfahren in die molekularbiologische Synthese mit hohem Reinheitsgrad unter Einschluß einer Bindungsstelle für spezifische Zelloberflächenrezeptoren zur Bindung an die Oberfläche bestimmter Zellen bzw. zur Zellmembranpassage, Einsatz gegen bösartige Tumore und deren Metastasierung.
15. Erzeugung von RiV-Subpartikeln durch an sich bekannte gentechnische und molekularbiologisch-biochemische Verfahren, d.h. Herstellung von RiV-Proteinen in unterschiedlichen Kombinationen und unterschiedlicher Modifikation und durch Verwendung von RiV-RNA oder anderen "Kondensationskemen", einschließlich zur schonenden Isolierung von RiV-Partikeln mit Hilfe spezifischer Affinität durch an sich bekannter Verfahren, z.B. Chromatographie, Hybridisierung.
16. Das akademische, letztlich auf Monokausal-Ätiologie (definiertes Agenz/definierte Erkrankung/Kausaltherapie) bewußt begrenzte Handlungsschema und das durch ungefilterten Leidensdruck erzwungene Handlungsschema der "Ganzheitsmedizin" (unbekanntes Agens/nicht definierte Erkrankung/empirische Therapie) können mittels BK-RiV-Behandlung - als schonende Substitutionstherapie im physiologischen Grenzbereich, d.h. als Ausgleich des mangelhaft funktionierenden AZSA - verbunden werden, da BK-RiV (Auslöser des AZSA) molekularbiologisch definiert, komplex und positiv in grundsätzliche pathologische Vorgänge eingreift, was ex juvantibus sowohl im einzelnen "Hilfe" auf bekanntem und unbekanntem Wege und grundsätzlich durch akademische Beobachtung "Erkenntnis" bedeutet. Dieser Gedankengang begründet eine breite Anwendung von BK-RiV in Medizin und Veterinärmedizin - ursprünglich als "ultima ratio" jetzt als "Mittel der Wahl".

## Patentansprüche

1. Verwendung von BK-RiV zur Herstellung von Präparaten für die Medizin und Veterinärmedizin zur Therapie und Prophylaxe von Zellerkrankungen durch molekularbiologisch bedingte, für das Gesamtindividuum schädliche (Fehl-) Informationen, die die Störung oder die Zerstörung des in der Evolution festgelegten Musters des jeweils betroffenen Organs durch pathologische Proliferation von veränderten Körperzellen verursachen und bei denen das "Allgemein zellulär-sekretorische Abwehrsystem" (AZSA) nicht in der Lage ist, den Vorgang in physiologischer Weise zu beenden, sowie zur Stabilisierung und Substitution des AZSA und der mit diesem kausal verknüpften Körperzustände, wobei die BK-RiV-Präparate
a) zur Behandlung von extremen Schmerzzuständen,
b) bei der Behandlung von Mykosen,
c) zur Behandlung von Psoriasis,
d) zur Behandlung von allergischen Erkrankungen,
e) zur Behandlung von Plasmozytom oder
f) zur Behandlung von Histiozytom (Sarkom)
eingesetzt werden.

2. Verwendung nach Anspruch 1,
**dadurch gekennzeichnet, dass**
die BK-RiV-Präparate mit Hilfe an sich bekannter Galenik als Externa-Präparate eingesetzt werden.

3. Verwendung nach Anspruch 1,
**dadurch gekennzeichnet, dass**
die BK-RiV-Präparate mit Hilfe an sich bekannter Galenik in Form von per os-Präparaten eingesetzt werden.

## Claims

1. Use of BK-RiV for the production of preparations to be used in medicine and veterinary medicine for the therapy and prophylaxis of cellular disorders as a result of (mis)information having molecular-biological causes and being harmful to the overall individual, which (mis)information causes interference with or destruction of the pattern established during evolution of each affected organ by pathological proliferation of modified body cells, and in case of which the "general cellular-secretory defense system" (GCDS) is not capable of terminating this process in a physiological fashion, and for the stabilization and substitution of the GCDS and of physical conditions causally associated with same, said BK-RiV preparations being used in
a) treatment of extreme conditions of pain,
b) treatment of mycotic infections,
c) treatment of psoriasis,
d) treatment of allergic diseases,
e) treatment of plasmocytoma, or
f) treatment of histiocytoma (sarcoma).

2. The use according to claim 1,
**characterized in that**
the BK-RiV preparations are employed as external preparations using per se known pharmaceutical technology.

3. The use according to claim 1,
**characterized in that**
the BK-RiV preparations are employed in the form of per os preparations using per se known pharmaceutical technology.

## Revendications

1. Utilisation de BK-RiV pour préparer des préparations pour la médecine et la médecine vétérinaire destinées à la thérapie et à la prophylaxie des maladies cellulaires dues à des informations nuisibles (fausses) à l'individu entier, par processus de biologie moléculaire, qui provoquent l'altération ou la destruction du modèle fixé dans l'évolution de l'organe respectivement concerné par une prolifération pathologique de cellules corporelles modifiées et pour lesquelles le 'système immunitaire sécrétoire cellulaire global' n'est pas en mesure de faire cesser le processus de manière physiologique, ainsi qu'à la stabilisation et à la substitution de ce système immunitaire sécrétoire cellulaire et des états corporels liés à celui-ci de manière causale, dans laquelle on utilise les préparations de BK-RiV
a) pour le traitement des états douloureux extrêmes,
b) pour le traitement des mycoses,
c) pour le traitement du psoriasis,
d) pour le traitement des maladies allergiques,
e) pour le traitement du plasmocytome ou
f) pour le traitement de l'histiocytome (sarcome).

2. Utilisation selon la revendication 1,
**caractérisée en ce**
**qu'**on utilise les préparations de BK-RiV en tant que préparation externe en utilisant une galénique connue pour cela.

3. Utilisation selon la revendication 1,
**caractérisée en ce**
**qu'**on utilise les préparations de BK-RiV sous forme de préparations per os en utilisant une galénique connue pour cela.
